# EUROPEAN PATENT SPECIFICATION

(11) **EP 1 558 139 B1**
(45) Date of publication and mention of the grant of the patent: **02.03.2011**
(21) Application number: 03777996.4
(22) Date of filing: 29.10.2003
(51) Int. Cl.: A61B 5/15

(54) **METHOD OF LANCING SKIN FOR THE EXTRACTION OF BLOOD**
VERFAHREN ZUR BLUTPROBEAUFNAHME MIT LANZETTE
PROCEDE POUR PIQUER LA PEAU DANS LE BUT D'EXTRAIRE DU SANG

(30) Priority: 30.10.2002 US 422228 P
(43) Date of publication of application: 03.08.2005
(60) Divisional application: 09075224.7
(73) Proprietor: LifeScan, Inc., Milpitas, CA 95035-6312 (US)
(72) Inventor: ALLEN, John, J., Mendota Heights, MN 55118 (US)
(74) Representative: Mercer, Christopher Paul
(86) International application number: PCT/US2003/034455
(87) International publication number: WO 2004/041087

## Description

### BACKGROUND OF THE INVENTION

### Field of the Invention

The present invention relates, in general, to an improved method of lancing skin and, more particularly, to an improved method wherein the lancing element is removed after creating an incision and then reinserted into the incision at a lesser depth of penetration.

### Background of the Invention

In in-situ testing of blood glucose, a glucose meter is placed against the skin and blood is sampled and measured without moving the meter. In one method of in-situ testing, a glucose sensor strip is combined with a lancing element positioned at a distal end thereof, the glucose sensor strip is then positioned in a meter adapted to launch the strip and lancing element combination toward the skin where the lancing element forms an incision. Blood or other bodily fluids such as, for example, interstitial fluid, may then be extracted from the incision and moved to the glucose sensor strip where it can be measured using, for example, an electrochemical process.

When lancing skin using an in-situ test strip, it is desirable to ensure that blood be transferred efficiently from the incision to the test strip, using as little blood as possible. Efficient transfer of blood from the incision means that more of the blood is actually used to test for analyte (e.g. glucose) levels, reducing the total blood required and, therefore, the incision size required for the test. Smaller incisions are particularly desirable because, in general, it is desirable to reduce the pain experienced by the user. Further, smaller incisions generally heal faster and are not as likely to re-open once healed.

Thus, when using an in-situ test, it is desirable to create an incision which is very small while maximizing the amount of blood generated by that incision. A number of factors influence the amount of blood generated by a particular incision. Many of those factors cannot be controlled. One of the factors which reduces the amount of blood available at a particular incision is the tendency of the incision to close and reseal after the lancing element is removed. Another factor which reduces the amount of blood available at a particular incision is the tendency of the wound to seal around the lancing element if the lancing element is left in the wound. Documents US 5,857,983 and EP 1157660 disclose prior art methods and devices for blood sampling.

It would, therefore, be advantageous to develop a method of lancing which increases the amount of blood available for testing at a particular incision site. It would further be advantageous to develop a method of lancing which increases the amount of blood available for lancing by preventing the wound from resealing during the testing process. It would further be advantageous to develop a method of lancing which increases the amount of blood available for lancing by preventing the wound from sealing around the lancing element during the testing process.

### SUMMARY OF THE INVENTION

In a method according to the present invention, a lancing tip is first inserted and then is retracted completely out of the lance wound site. The lancing tip is then re-inserted into the same wound to a depth which is shallower than the original penetration. This method of lancing facilitates the expression of blood and its seamless transfer into a test strip integrated with a lance.

In a method of lancing skin according to the present invention, the lancing element is inserted through an outer surface of the skin to obtain a sample of blood. In one embodiment of the invention, the method includes using a lancing instrument having a sharpened end and a fluid channel extending from the sharpened end to a sensor attached to a proximal end of the lancing instrument, forcing the sharpened tip of the lancing element into the skin to a first predetermined depth, wherein the sharpened tip creates an incision in the skin surface and a wound below the skin surface and completely withdrawing the lancing element, including the sharpened tip. After completely withdrawing the sharpened tip from the incision, sharpened tip is forced back into the incision to a second predetermined depth, wherein the second predetermined depth is not as deep as the first predetermined depth. Once the sharpened tip is positioned at the second predetermined depth, blood is drawn through the channel to the sensor.

In a method of lancing skin in accordance with the present invention, as set forth above, the method may further include ensuring that the second predetermined depth is sufficient to ensure that a distal end of the channel is positioned below the surface of the skin. For example, in one embodiment of the invention, the method may include setting the first predetermined depth in the range of approximately 0.25 to 1.5 mm. Further, in one embodiment of the invention, the method may include setting the second predetermined depth is in the range of approximately 0.05 to 0.25mm.

In a method of lancing skin according to the present invention, the lancing element is inserted through an outer surface of the skin to obtain a sample of blood. In one embodiment of the invention, the method includes providing pressure on the skin in a region surrounding the incision site (i.e. the site where the incision is to be made). Then using a lancing instrument having a sharpened end and a fluid channel extending from the sharpened end to a sensor attached to a proximal end of the lancing instrument, forcing the sharpened tip of the lancing element into the skin to a first predetermined depth, wherein the sharpened tip creates an incision in the skin surface and a wound below the skin surface and completely withdrawing the lancing element, including the sharpened tip. After completely withdrawing the sharpened tip from the incision, sharpened tip is forced back into the incision to a second predetermined depth, wherein the second predetermined depth is not as deep as the first predetermined depth. Once the sharpened tip is positioned at the second predetermined depth, blood is drawn through the channel to the sensor.

In a method according to the present invention, a lancing tip is extended beyond an opening at a distal end of a lancing device, such as, for example, an in-situ meter. The lancing tip is extended to a first extension length beyond the opening to create a wound and then retracted past the opening. The lancing tip is then re-extended past the opening to a second extension length which is less than the first extension length and blood is drawn up the lancing element from the wound into a test strip integrated with a lance.

In a method of lancing skin according to the present invention, the lancing element is extended a first distance past an opening and through an outer surface of the skin to obtain a sample of blood. In one embodiment of the invention, the method includes using a lacing instrument having a sharpened end and a fluid channel extending from the sharpened end to a sensor attached to a proximal end of the lancing instrument, extending the sharpened tip a first extension length beyond an opening, forcing the sharpened tip of the lancing element into the skin, wherein the sharpened tip creates an incision in the skin surface and a wound below the skin surface, withdrawing the lancing element into the opening and extending the sharpened tip a second extension length beyond the opening, wherein the second extenstion depth is less than the first extension depth. Once the sharpened tip is positioned at the second extension depth, blood is drawn through the channel to the sensor.

In a method of lancing skin in accordance with the present invention, as set forth above, the method may further include ensuring that the extension length is sufficient to ensure that a distal end of the channel is positioned below the surface of the skin. For example, in one embodiment of the invention, the method may include setting the first extension length in the range of approximately 0.25 to 1.5 mm. Further, in one embodiment of the invention, the method may include setting the second extension length is in the range of approximately 0.05 to 0.25mm.

In a method of lancing skin according to the present invention, the lancing element is inserted through an outer surface of the skin to obtain a sample of blood. In one embodiment of the invention, the method includes providing pressure on the skin in a region surrounding the incision site (i.e. the site where the incision is to be made). Then using a lancing instrument having a sharpened end and a fluid channel extending from the sharpened end to a sensor attached to a proximal end of the lancing instrument, forcing the sharpened tip of the lancing element though an opening into the skin to a first extension length beyond the opening, wherein the sharpened tip creates an incision in the skin surface and a wound below the skin surface and completely withdrawing the lancing element, including the sharpened tip into the opening. After completely withdrawing the sharpened tip into the opening, extending the sharpened tip through the opening and into the incision to a second extension length beyond the opening, wherein the second extension length is not as long as the first extension length. Once the sharpened tip is positioned at the second extension length, blood is drawn through the channel to the sensor.

In a method of lancing skin in accordance with the present invention, as set forth above, the method may further include providing a milking ring wherein the pressure in the region surrounding the incision site is exerted by the milking ring. The milking ring is positioned on the skin prior to the step of forcing the sharpened tip into the skin and may be maintained throughout the remainder of the procedure. In this embodiment of the invention, the milking ring provides a pressure sufficient to facilitate the flow of bodily fluids into the channel after the reinsertion of the lancing tip into the wound. In one embodiment of the invention, the milking ring provides a pressure in a range of approximately 0.5 to 1.5 pounds. In a further embodiment of the present invention, the method may include positioning the milking ring against the skin for a predetermined period of time prior to launching the lancing element. In a further embodiment of the present invention, the predetermined period of time may be three seconds or more.

### BRIEF DESCRIPTION OF THE DRAWINGS

The novel features of the invention are set forth with particularity in the appended claims. A better understanding of the features and advantages of the present invention will be obtained by reference to the following detailed description that sets forth illustrative embodiments, in which the principles of the invention are utilized, and the accompanying drawings of which:
Figure 1 is a perspective view of a lancing element and strip for use in a method according to the present invention.
Figure 2 is a perspective view of the top layer of a lancing element and strip for use in a method according to the present invention.
Figure 3A is a perspective view of a lancing element tip immediately before penetration of she surface of the skin.
Figure 3B is a side view of the lancing element illustrated in Figure 3A.
Figure 3C is a perspective view of a lancing element as the tip of the lancing element begins to penetrate the surface of the skin.
Figure 3D is a side view of the lancing element as the tip of the lancing element begins to penetrate the surface of the skin.
Figure 3E is a perspective view of the lancing element as it reaches its full depth of penetration beneath the surface of the skin.
Figure 3F is a side view of the lancing element as it reaches its full depth of penetration beneath the surface of the skin.
Fig 3G is a perspective view of the lancing element after it is fully withdrawn from the skin.
Fig 3H is a side view of the lancing element after it is full withdrawn from the skin.
Figure 3I is a perspective view of the lancing element as it re-enters the surface of the skin through the incision created during the first entry.
Figure 3J is a side view of the lancing element as it re-enters the surface of the skin through the incision created during the first entry.
Figure 3K is a perspective view showing the lancing element being used to draw blood from a forearm.

### DETAILED DESCRIPTION OF ILLUSTRATIVE EMBODIMENTS OF THE INVENTION

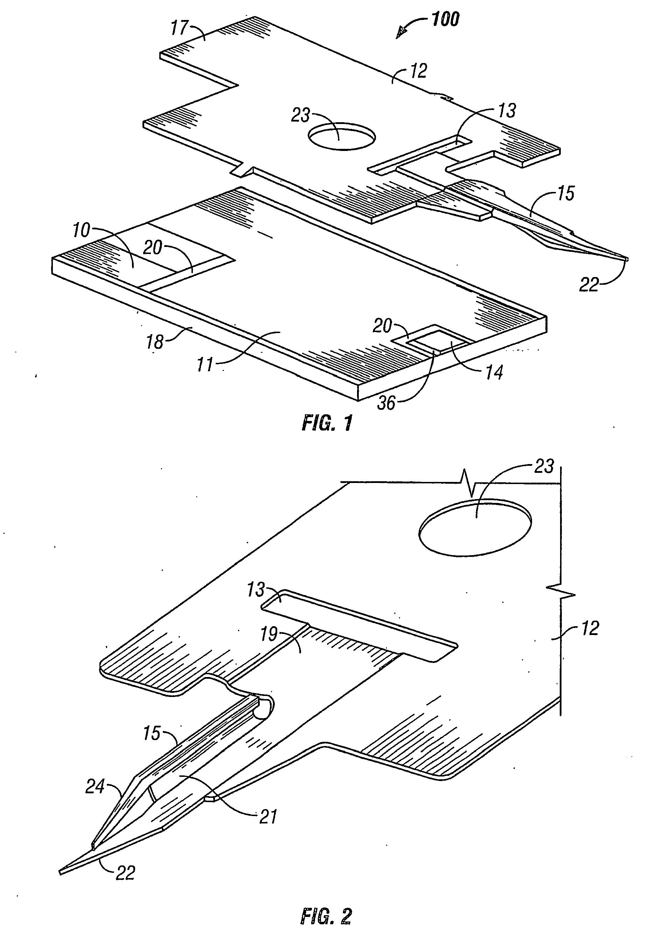

Figure 1 is a perspective view of a lancing element and strip for use in a method according to the present invention. In Figure 1, lancing element 15 is connected to sensor strip 100. Sensor strip 100 may be, for example, a glucose sensor strip which uses electrochemistry to measure the amount of glucose in a bodily fluid, such as, for example, blood or interstitial fluid. In Figure 1, lancing element further includes lancing tip 22. Sensor strip 100 further includes first electrode contact 10, adhesive layer 11, conductive substrate 12, vent hole 13, analyte sensing layer 14, second electrode contact 17, insulating substrate 18, insulating layer 20, registration hole 23 and working electrode 36.

Figure 2 is a perspective view of the top layer of a lancing element and strip for use in a method according to the present invention. In Figure 2 top layer is formed of conductive substrate 12. In the embodiment illustrated in Figure 2, conductive substrate 12 includes vent hole 13 and registration hole 23. In Figure 2, lancing element includes lancing tip 22, channel tip 24 and fill channel 21.

One embodiment of a lancing element and sensor strip suitable for use in a method according to the present invention may be described with reference to Figures 1 and 2. In the embodiment illustrated in Figures 1 and 2, sensor strip 100 includes first electrode contact 10, wherein first electrode contact may be screen printed on an insulating substrate 18, and a second electrode contact 17, wherein said second electrode contact comprises a portion of conductive substrate 12 which is contiguous with top reference electrode 19 and lancing element 15.

In the embodiment of the lancing element and sensor strip illustrated in Figures 1 and 2, the orientation of said first electrode contact 10 and second electrode contact 17 are arranged such that an analyte measurement meter, such as, for example, a glucose meter (not shown) can establish electrical contact with sensor strip 100. In the illustrated embodiment, the electrodes are arranged on the same side of insulating substrate 18 to facilitate contact of both electrodes at the proximal end of sensor strip 100.

Sensor strip 100 is manufactured using adhesive layer 11 to attach insulating substrate 18 to conductive substrate 12. Adhesive layer 11 could be implemented in a number of ways, including using pressure sensitive material, heat activated material, or UV cured double sided adhesive material. Conductive substrate 12 may be, for example, a conductive substrate that is a sheet of electrically conductive material such as gold or plated stainless steel. The geometry of conductive substrate 12 may be formed by, for example, stamping process or photo-etching. In the embodiment illustrated in Figures 1 and 2, lancing element 15 may be manufactured as an integral part of conductive substrate 12. Vent hole 13, may be formed by, for example, punching through conductive layer 12. Vent hole 13 is used to facilitate the transport of bodily fluid up lancing element 15 and across analyte sensing layer 14. Registration hole 23 may be formed during the stamping process of making conductive substrate 12.

In one embodiment of the invention, analyte sensing layer 14 may be, for example, a glucose sensing layer, including an enzyme, a buffer, and a redox mediator. Analyte sensing layer 14 may preferably be deposited on top of working electrode 36. Where analyte sensing layer 14 is used to detect the presence and concentration of glucose in a bodily fluid, at least a portion of glucose sensing layer 14 dissolves in the bodily fluid and is used to convert the glucose concentration into an electrically measured parameter which is proportional to the glucose concentration in the sample.

In the embodiment illustrated in Figures 1 and 2, lancing element 15 has a proximal and distal end and the proximal end is integrated with the top reference electrode 19 and said distal end is integrated with a.lancing tip 22 and channel tip 24. The lancing element is formed by the process of stamping or photo-etching a conductive material sheet and bending it to the geometry shown in Figure 2. In one embodiment, lacing tip 22 and channel tip 24 are slightly offset by about 0.005 to 0.020", the design of lancing element 15 is adapted-to assist in improving skin separation. The geometry illustrated in Figures 1 an 2 may enhance fluid egress because it helps spread and hold open a skin wound. In the embodiment illustrated in Figures 1 and 2, the lancing element 15 is contiguous with the top reference electrode 19 and electrode contact 17.

In the embodiment of the invention illustrated in Figure 2, lancing element 15 includes fill channel 21, wherein capillary fill channel 21 facilitates the flow of body fluid from the wound to the analyte sensing layer 14. Fill channel 21 may facilitate the flow of bodily fluids by, for example, wicking or capillary action. In the embodiment illustrated in Figures 1 and 2 fill channel 21 has an open geometry which facilitates the wicking of viscous samples and provides for simpler manufacturing techniques when compared with closed capillary channels.

In the embodiment of sensor strip 100 illustrated in Figure 1, insulating substrate 18 consists of material such as polyester or ceramic on which a conductive material can be printed onto the insulating layer through silk-screening, sputtering, or electro-less deposition. Conductive material deposited on insulating substrate 18 forms first electrode contact 10 and working electrode 36. Insulating layer 20 may be, for example, screen printed to form a boundary for the electrode contact 10 and the bottom working electrode.

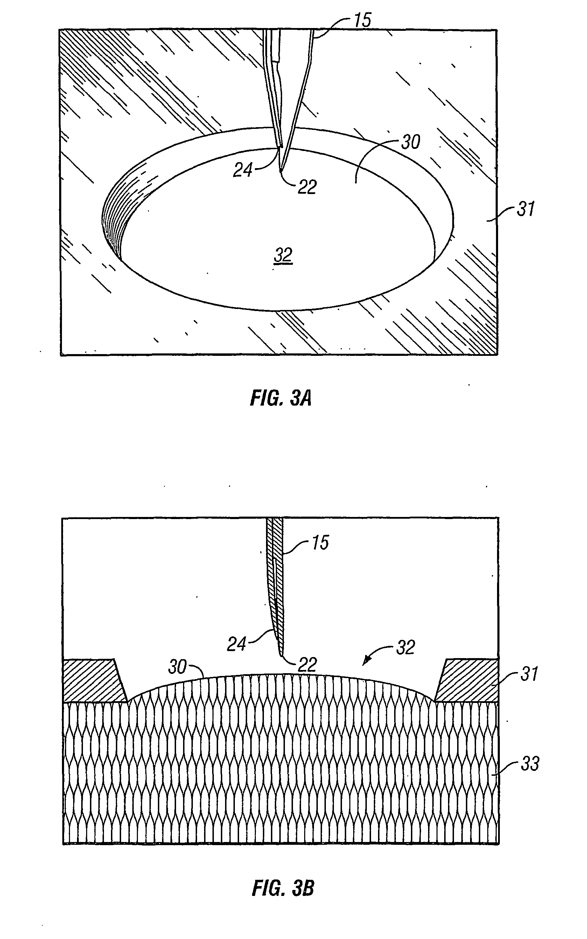

Figure 3A is a perspective view of a lancing element tip immediately before penetration of the surface of the skin. More particularly, Figure 3A is a perspective view of a lance 15 before lancing tip 22 penetrates skin surface 30. Figure 3B is a side view of the lancing element illustrated in Figure 3A. More particularly Figure 3B is side view of lancing element 15 before lancing tip 22 penetrates skin surface 30. In Figures 3A and 3B, milking ring 31 is placed against skin surface 30, causing skin surface 30 to bulge into milking opening 32. Opening 32 may further be the opening at the distal end of a lancing device, such as, for example, an in-situ meter wherein the lancing element 15 extends through opening 32 to puncture skin. Milking ring 32 may be, for example, a substrate with a hole drilled through it which could be, for example, a plastic such as polystyrene, polyethylene, polycarbonate, polyester, or the like. The diameter of opening 32 of said milking ring may be, for example, in the range of between 3.5 and 12 mm. In operation, the milking ring 31 may be applied with gentle pressure onto a fingertip, forearm, or other suitable site such that the skin surface 30 forms a raised mound within milking ring 31. In a one embodiment of a method according to the present invention, milking ring 31 is applied to skin surface 31 with a pressure of approximately 0.5 to 1.5 pounds of applied pressure. In one embodiment of a method according to the present invention, the use of a milking ring is intended to facilitate the collection of bodily fluids by applying a pressure around the incision site to provide a driving force for expressing fluid from the wound site.

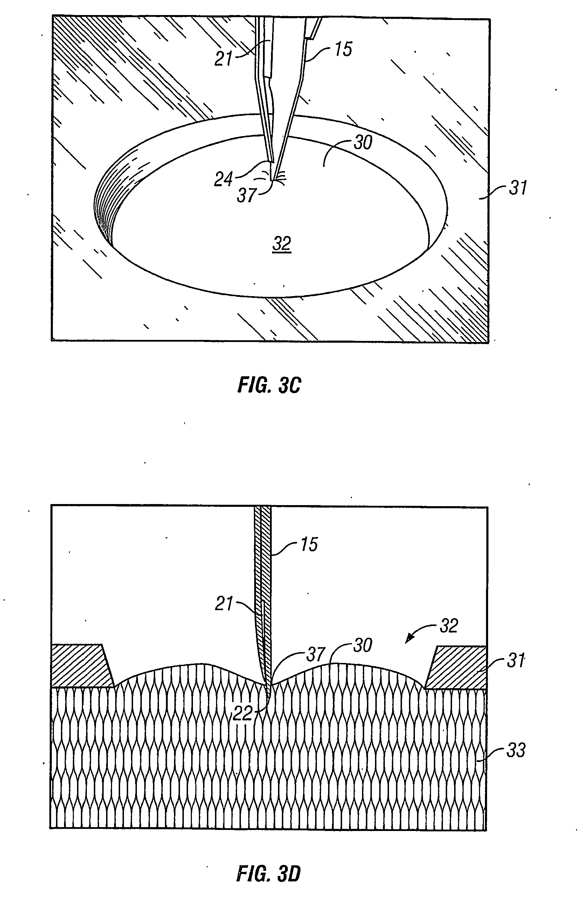

Figure 3C is a perspective view of a lancing element as the tip of the lancing element begins to penetrate the surface of the skin. As lancing tip 22 enters skin surface 30, deflecting skin surface 30 away from lancing tip 22 until skin surface 30 is punctured, forming an incision 37 in skin surface 30, enabling lancing element 15 to enter subcutaneous region 33. Figure 3D is a side view of the lancing element as the tip of the lancing element begins to penetrate the surface of the skin. More particularly, Figure 3D is a side view of lancing element 15 as lancing tip 22 enters incision 37 in skin surface 30.

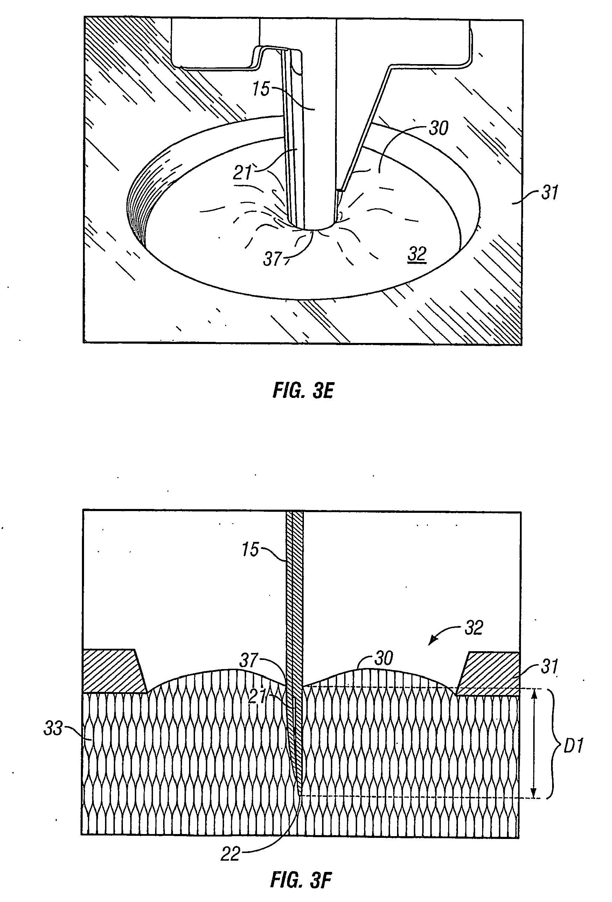

Figure 3E is a perspective view of the lancing element as it reaches its full depth of penetration beneath the surface of the skin. In one embodiment of the present invention, the penetration depth may be reached by extending the lancing tip 22 to a first extension length beyond opening 32. More particularly, Figure 3E is a perspective view of lancing element 15 after lancing tip 22 has reached its full depth of penetration into subcutaneous region 33. Figure 3F is a side view of the lancing element 15 as it reaches its full depth of penetration beneath the surface of the skin. More particularly, Figure 3F is a side view of lancing element 15 after lancing tip 22 has reached its full depth of penetration into subcutaneous region 33. At full penetration, lancing tip 22 reaches a depth of D1. The actual value of D1 for a particular application will depend upon a number of factors, including the bodily fluid being extracted. For example, if the bodily fluid being extracted is blood, the depth D1 will be greater than if the bodily fluid being extracted is interstitial fluid (i.e. ISF). In one embodiment of the present invention, penetration depth D1 may be, for example, in the range of 0.25 to 1.5 mm deep. In a further embodiment of the present invention, D1 may represent the first extension length measured from lancing tip 22 to opening 32..

In a method according to the present invention, insertion of lancing element 15 through skin surface 30 creates an incision 37 in addition to severing subcutaneous tissue and capillaries and providing fill channel 21 with access to the bodily fluid to be sampled, whether blood or interstitial fluid. Thus, with lancing element 15 positioned as shown in Figures 3E and 3F, bodily fluid will flow through fill channel 21 and into sensor strip 100. However, leaving lancing element 15 positioned as illustrated in Figures 3E and 3F will not provide an optimal flow of bodily fluid through fill channel 21. The reasons for the limited flow may include, for example, the blocking of lanced capillaries by the location of lancing element 15 which may, for example, prevent the capillaries or interstitial fluid from flowing freely and pooling in the wound created by lancing element 15.

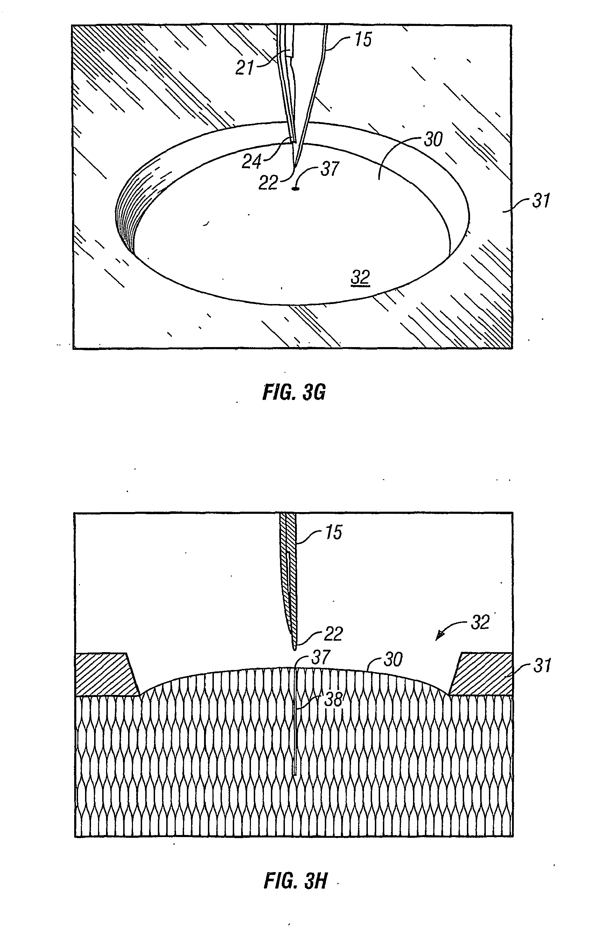

Fig 3G is a perspective view of the lancing element after it is fully withdrawn from the skin. More particularly, Figure 3G is a perspective view of lancing element 15 after lancing tip 22 has been withdrawn completely from incision 37. Fig 3H is a side view of the lancing element 15 after it is fully withdrawn from skin surface 30. In a further embodiment of the present invention, lancing tip 22 is withdrawn fully past opening 32. In a method according to the present invention, fully withdrawing lancing element 15 from incision 37 creates an open wound 38 below incision 37 that facilitates expression of bodily fluid into wound 38. By fully removing lancing element 15 from the wound in accordance with the method of the present invention, bodily fluids flow more readily into wound 38. If lancing element 15 is not completely removed from wound 38, blood and/or interstitial fluid flow may be impeded. One possible explanation for the limited blood flow is the possibility that the partially retracted lancing element 15 may still effectively block severed capillaries because of the resiliency of the skin. After the initial skin stretching during the penetration event, the skin might revert back to its initial position around lancing element 15. Thus, in the method according to the present invention it is important that lancing element 15 be fully removed from wound 38 after the initial penetration to allow bodily fluid to pool in wound 38.

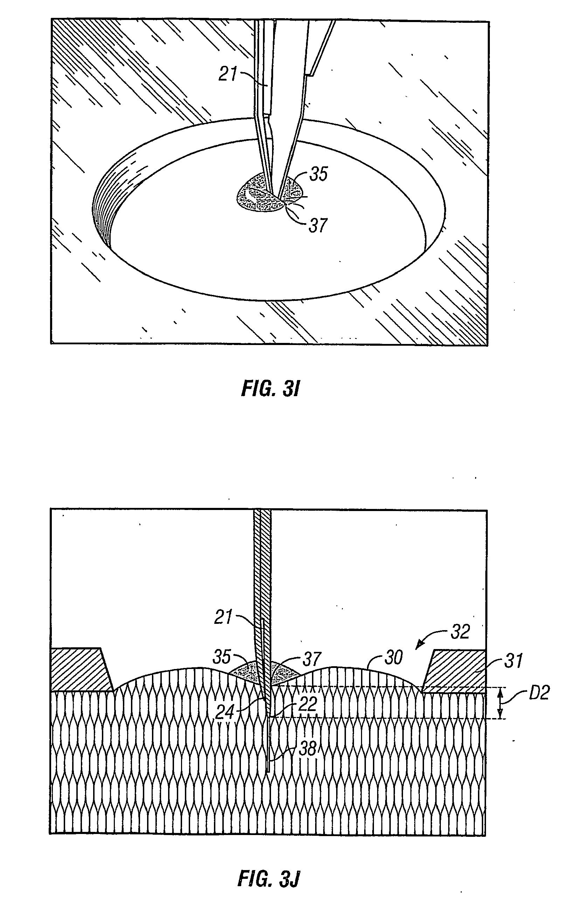

Figure 3I is a perspective view of the lancing element as it re-enters the surface of the skin through the incision created during the first entry. More particularly, Figure 3I is a perspective view of lancing element 15 after lancing tip 22 has been re-inserted through incision 37 into wound 38. Figure 3J is a side view of the lancing element as it re-enters the surface of the skin through the incision created during the first entry. More particularly, Figure 3J is a side view of lancing element 15 positioned within wound 38 such that channel tip 24 is below skin surface 30. In a method according to the present invention, illustrated in Figure 3J, lancing tip 22 is reinserted to a depth D2 which is less than D1. In a further embodiment of the present invention, D2 may represent a second extension length, wherein lancing tip 22 is extended past opening 32 to second extension length D2, in this embodiment of the invention, wherein second extension length D2 is less than first extension length D1. Partially reinserting in accordance with a method according to the present invention facilitates the flow of bodily fluids into fluid channel 21. In one embodiment of the present invention, the distance D2 to which lancing tip 22 penetrates is generally defined by the distance from lancing tip 22 to channel tip 22. In one embodiment of the invention, lancing tip 22 may be reinserted to a depth D2 of approximately 0.05 to 0.25 mm deep, wherein D2 is defined as the distance from a surface of the skin 34 to the lancing tip 22.

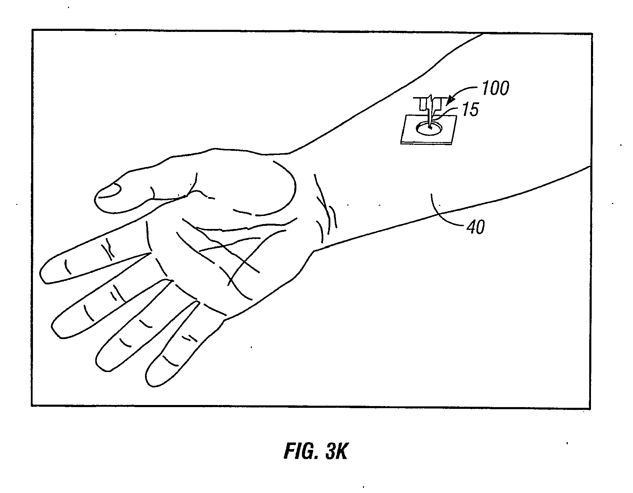

Figure 3K is a perspective view showing the lancing element being used to draw blood from a forearm. More particularly, Figure 3K is a perspective view of a lancing device 15 being used in a method according to the present invention to draw bodily fluids from a forearm 40 of a human being.

In a method of lancing skin in accordance with the present invention, as set forth above, the method may further include using milking ring 31 to exert the pressure in the region surrounding incision 37 exerted by milking ring 31. Milking ring 31 is positioned on the skin prior to the step of forcing the lancing tip 22 into the skin and may be maintained throughout the remainder of the procedure. In this embodiment of the invention, the milking ring 31 provides a pressure sufficient to facilitate the flow of bodily fluids into fill channel 21 after the reinsertion of lancing tip 22 into wound 38. In one embodiment of the invention, milking ring 31 provides a pressure in a range of approximately 0.5 to 1.5 pounds. In a further embodiment of the present invention, the method may include positioning the milking ring 31 against the skin for a predetermined period of time prior to launching the lancing element. In a further embodiment of the present invention, the predetermined period of time may be three seconds or more.

It will be recognized that equivalent structures may be substituted for the structures illustrated and described herein and that the described embodiment of the invention is not the only structure which may be employed to implement the claimed invention. As one example of an equivalent structure which may be used to implement the present invention, a lancing element may be used which does not include a channel tip, with the channel extending from the distal end of the lancing element to the working electrode. While preferred embodiments of the present invention have been shown and described herein, it will be obvious to those skilled in the art that such embodiments are provided by way of example only. Numerous variations, changes, and substitutions will now occur to hose skilled in the art without departing from the invention. It should be understood that various alternatives to the embodiments of the invention described herein may be employed in practicing the invention. It is intended that the following claims define the scope of the invention and that methods and structures within the scope of these claims and their equivalents be covered thereby.

## Claims

1. A method of lancing skin through an outer surface of said skin to obtain a sample of blood, the method comprising the steps of:
providing a lancing instrument having a sharpened tip and a channel adjacent said sharpened tip to a sensor attached to a proximal end of said lancing instrument;
forcing said sharpened tip into said skin to a first predetermined depth below said outer surface, wherein said sharpened tip creates an incision in said skin surface;
completely with drawing said sharpened tip from said incision;
forcing said sharpened lip back into said incision to a second predetermined depth, wherein said second predetermined depth is not as deep as said first predetermined depth; and
drawing blood through said channel to said sensor.

2. A method of lancing skin as set forth in Claim 1 wherein said second predetermined depth is sufficient to insure that a distal end of said channel is positioned below said surface of said skin.

3. A method of lancing skin as set forth in Claim 1 wherein said fust predetermined depth is in the range of approximately 0.25 to 1.5 mm,

4. A method of lancing skin as set forth in Claim 3 wherein said second predetermined depth is in the range of approximately 0.05 to 0.25mm.

5. A method of lancing skin through an outer surface of said skin to obtain a sample of blood, the method comprising the steps of:
providing a lancing instrument having an opening and a lancing element having a sharpened tip and a channel extending from adjacent said sharpened tip to a sensor attached to a proximal end of said lancing element;
forcing said sharpened tip into said skin by extending said sharpened tip to a first extension length beyond said opening, wherein said sharpened tip creates an incision in said skin surface;
completely withdrawing said sharpened tip from said incision;
forcing said sharpened tip back into said incision by extending said sharpened tip to a second extension length beyond said opening, wherein said second extension length is less than said first extension length; and
drawing blood through said channel to said sensor.

6. A method of lancing skin as set forth in Claim 5 wherein said second extension length is sufficient to inspire that a distal end of said channel is positioned below said surface of said skin.

7. A method of lancing skin as set forth in Claim 5 wherein said first extension length is in the range of approximately 0.25 to 1.5 mm.

8. A method of lancing skin as set forth in Claim 7 wherein said second extension length is in the range of approximately 0.05 to 0.25mm.

9. A method of lancing skin as set forth in Claim 1, or Claim 5, further comprising the step of providing pressure on said skin in a region surrounding said incision.

10. A method of lancing skin as set forth in Claim 9 wherein said pressure is exerted by a milking ring positioned on said skin prior to said step of forcing said sharpened tip into said skin.

11. A method of lancing skin as set forth in Claim 10 wherein said milking ring provides a pressure sufficient to facilitate the flow of bodily fluids into said channel.

12. A method of lancing skin as set forth in Claim 11 wherein said milking ring provides a pressure in a range of approximately 0.5 to 1.5 pounds.

13. A method of lancing skin as set forth in Claim 1 or Claim 5, wherein said second predetermined depth is sufficient to insure that a distal end of said channel is positioned adjacent said surface of said skin.

14. A method of lancing skin as set forth in Claim 9 where in said pressure is applied for a predetermined time period prior to lancing said skin, said predetermined time period being approximately three seconds or more.

## Patentansprüche

1. Verfahren zum Aufstechen von Haut durch eine Außenfläche der Haut, um eine Blutprobe zu erhalten, wobei das Verfahren die Schritte aufweist:
Bereitstellen eines Stechinstrumentes mit einer geschärften Spitze und einem Kanal benachbart der geschärften Spitze zu einem Sensor, der an einem proximalen Ende des Stechinstrumentes angebracht ist;
Treiben der geschärften Spitze in die Haut in eine erste vorab festgelegte Tiefe unterhalb der Außenfläche, wobei die geschärfte Spitze eine Inzision in der Hautfläche erzeugt;
vollständiges Zurückziehen der geschärften Spitze aus der Inzision;
Treiben der geschärften Spitze zurück in die Inzision in eine zweite vorab festgelegten Tiefe, wobei die zweite vorab festgelegte Tiefe nicht so tief ist wie die erste vorab festgelegte Tiefe; und
Ziehen von Blut durch den Kanal zu dem Sensor.

2. Verfahren zum Aufstechen von Haut nach Anspruch 1, bei dem die zweite vorab festgelegte Tiefe ausreichend ist, sicherzustellen, dass ein distales Ende des Kanals unter der Fläche der Haut positioniert ist.

3. Verfahren zum Aufstechen von Haut nach Anspruch 1, bei dem die erste vorab festgelegte Tiefe in dem Bereich von ungefähr 0.25 bis 1.5 mm liegt.

4. Verfahren zum Aufstechen von Haut nach Anspruch 3, bei dem die zweite vorab festgelegte Tiefe in dem Bereich von ungefähr 0.05 bis 0.25 mm liegt.

5. Verfahren zum Aufstechen von Haut durch eine Außenfläche der Haut, um eine Blutprobe zu erhalten, wobei das Verfahren die Schritte aufweist:
Bereitstellen eines Stechinstrumentes, das eine Öffnung und ein Stechelement mit einer geschärften Spitze und einem Kanal, der sich von benacht der geschärften Spitze zu einem Sensor, der an einem proximalen Ende des Stechelementes angebracht ist, erstreckt, aufweist;
Zwingen der geschärften Spitze in die Haut durch Ausfahren der geschärften Spitze zu einer ersten Ausfahrlänge über die Öffnung hinaus, wobei die geschärfte Spitze eine Inzision in der Hautfläche erzeugt;
vollständiges Zurückziehen der geschärften Spitze aus der Inzision;
Zwingen der geschärften Spitze zurück in die Inzision durch Ausfahren der geschärften Spitze zu einer zweiten Ausfahrlänge über die Öffnung hinaus, wobei die zweite Ausfahrlänge geringer ist als die erste Ausfahrlänge; und
Ziehen von Blut durch den Kanal zu dem Sensor.

6. Verfahren zum Aufstechen von Haut nach Anspruch 5, wobei die zweite Ausfahrlänge ausreichend ist, um sicherzustellen, dass ein distales Ende des Kanals unter der Fläche der Haut angeordnet ist.

7. Verfahren zum Aufstechen von Haut nach Anspruch 5, bei dem die erste Ausfahrlänge in dem Bereich von ungefähr 0.25 bis 1.5 mm liegt.

8. Verfahren zum Aufstechen von Haut nach Anspruch 7, bei dem die zweite Ausfahrlänge in dem Bereich von ungefähr 0.05 bis 0.25 mm liegt.

9. Verfahren zum Aufstechen von Haut nach Anspruch 1 oder Anspruch 5, das weiter den Schritt des Ausübens von Druck auf die Haut in einem Bereich, der die Inzision umgibt, aufweist.

10. Verfahren zum Aufstechen von Haut nach Anspruch 9, bei dem der Druck durch einen Pressring, der auf der Haut angeordnet wird, ausgeübt wird, vor dem Schritt des Zwingens der geschärften Spitze in die Haut.

11. Verfahren zum Aufstechen von Haut nach Anspruch 10, bei dem der Pressring für einen Druck sorgt, der ausreichend ist, um den Fluss von Körperfluiden in den Kanal zu erleichtern.

12. Verfahren zum Aufstechen von Haut nach Anspruch 11, bei dem der Pressring einen Druck in einem Bereich von ungefähr 0.5 bis 1.5 Pfund liefert.

13. Verfahren zum Aufstechen von Haut nach Anspruch 1 oder Anspruch 5, bei dem die zweite vorab festgelegte Tiefe ausreichend ist, um sicherzustellen, dass ein distales Ende des Kanals benachbart der Fläche der Haut angeordnet ist.

14. Verfahren zum Aufstechen von Haut nach Anspruch 9, bei dem der Druck über eine vorab festgelegte Zeitdauer vor dem Aufstechen der Haut ausgeübt wird, wobei die vorab festgelegte Zeitdauer ungefähr drei Sekunden oder mehr beträgt.

## Revendications

1. Système pour retirer du fluide corporel d'une région d'une partie du corps, en particulier d'un bout de doigt, comprenant:
- une unité de compression contre laquelle la partie du corps est pressée dans une direction primaire (82) et convertit partiellement la pression appliquée en un mouvement dans une direction secondaire (84, 84') avec une composante perpendiculaire à la direction primaire de telle sorte que la pression interne est augmentée dans une région de la partie de corps,
- un dispositif de perforation, en particulier une lancette ou canule, pour produire une ouverture de corps dans la zone de la pression interne accrue,
où l'unité de compression renferme au moins partiellement la partie de corps, en particulier un doigt, lorsque la partie de corps est pressée contre celle-ci.

2. Système selon la revendication 1, dans lequel l'unité de compression possède une région en forme de U (80) dont les parties latérales se déplacent l'une vers l'autre lorsqu'une partie de corps est pressée contre celle-ci.

3. Système selon la revendication 2, dans lequel la région en forme de U (80) est flexible, et les extrémités de celle-ci sont reliées à des leviers (81, 81') d'une longueur essentiellement constante.

4. Système selon la revendication 2, dans lequel la région en forme de U est formée par deux moitiés de coque essentiellement inflexibles (80a, 80b) qui sont assemblées par une région flexible (85).

5. Système selon la revendication 1, dans lequel l'unité de compression possède un matériau (90) qui est déformable mais possède un volume essentiellement constant et est agencée dans un châssis (91).

6. Système selon la revendication 2, dans lequel l'unité de compression est fabriquée à partir d'une mousse en plastique ou en caoutchouc.

7. Système selon la revendication 1, dans lequel le dispositif de perforation est disposé d'une manière déplaçable relativement à l'unité de compression.

8. Système selon la revendication 7, dans lequel le dispositif de perforation est chargé par ressort.

9. Système selon les revendications 1, 7 ou 8, dans lequel la profondeur de ponction du dispositif de perforation est ajustable.

10. Système selon la revendication 1, dans lequel un système analytique pour la détermination de la concentration d'un analyte, en particulier du glucose, est intégré.

11. Système selon la revendication 9, dans lequel une unité de commande est intégrée, qui commande d'une manière coordonnée l'activation du dispositif de perforation et du système analytique.

12. Système selon la revendication 9, dans lequel le système analytique contient au moins un élément de test.

13. Système pour stimuler l'écoulement d'un fluide corporel d'une partie de corps, en particulier du bout de doigt, comprenant une unité de compression qui convertit partiellement une pression appliquée par la partie de corps dans une direction primaire en un mouvement dans une direction secondaire avec une composante à angles droits à la direction primaire et augmente ainsi la pression interne dans une région de la partie de corps où l'unité de compression renferme au moins partiellement la partie de corps lorsqu'elle est pressée contre celle-ci.

14. Procédé de stimulation de l'écoulement du fluide corporel d'une partie de corps, en particulier du bout du doigt, dans lequel la partie de corps est pressée contre une unité de compression qui convertit partiellement le mouvement de pression primaire en un mouvement dans une direction secondaire transversale à la direction primaire de sorte que la pression interne est augmentée dans une région de la partie de corps où l'unité de compression renferme au moins partiellement la partie de corps lorsqu'elle est pressée contre celle-ci.
